# EUROPÄISCHE PATENTSCHRIFT

(11) **EP 1 409 727 B1**
(45) Veröffentlichungstag und Bekanntmachung des Hinweises auf die Patenterteilung: **09.11.2005**
(21) Anmeldenummer: 02732726.1
(22) Anmeldetag: 17.05.2002
(51) Int. Cl.: C12Q 1/68

(54) **VERFAHREN ZUM QUALITATIVEN UND/ODER QUANTITATIVEN NACHWEIS VON ZELLEN**
METHOD FOR QUALITATIVE AND/OR QUANTITATIVE DETECTION OF CELLS
PROCEDE DESTINES A LA DETECTION QUALITATIVE ET/OU QUANTITATIVE DE CELLULES

(30) Priorität: 06.09.2001 DE 10143691; 06.09.2001 DE 10143699; 06.09.2001 DE 10143775; 06.09.2001 DE 10143776; 22.11.2001 WO PCT/EP01/13606
(43) Veröffentlichungstag der Anmeldung: 21.04.2004
(73) Patentinhaber: Adnagen AG, 30853 Hannover-Langenhagen (DE)
(72) Erfinder: ALBERT, Winfried, 82362 Weilheim (DE); STEFFENS, Pia, D- 30177 Hannover (DE); KREHAN, Alf-Andreas, D- 85646 Anzing (DE); WASCHÜTZA, Stefanie, 30169 Hannover (DE)
(74) Vertreter: Pfenning, Meinig & Partner GbR
(86) Internationale Anmeldenummer: PCT/EP2002/005489
(87) Internationale Veröffentlichungsnummer: WO 2003/023057

(56) Entgegenhaltungen:
- WO-A-94/07139
- WO-A-96/29430
- WO-A-97/37226
- WO-A-98/12227
- WO-A-99/44064
- DE-A- 19 736 691
- US-B1- 6 187 546
- HARDINGHAM J E ET AL: "IMMUNOBEAD-PCR: A TECHNIQUE FOR THE DETECTION OF CIRCULATING TUMOR CELLS USING IMMUNOMAGNETIC BEADS AND THE POLYMERASE CHAIN REACTION" CANCER RESEARCH, AMERICAN ASSOCIATION FOR CANCER RESEARCH, BALTIMORE, MD, US, Bd. 53, 1. August 1993 (1993-08-01), Seiten 3455-3458, XP000605489 ISSN: 0008-5472
- NACHT MARIANA ET AL: "Combining serial analysis of gene expression and array technologies to identify genes differentially expressed in breast cancer" CANCER RESEARCH, AMERICAN ASSOCIATION FOR CANCER RESEARCH, BALTIMORE, MD, US, Bd. 59, Nr. 21, 1. November 1999 (1999-11-01), Seiten 5464-5470, XP002166291 ISSN: 0008-5472
- REN-HEIDENREICH L ET AL: "SPECIFIC TARGETING OF EGP-2+ TUMOR CELLS BY PRIMARY LYMPHOCYTES MODIFIED WITH CHIMERIC T CELL RECEPTORS" HUMAN GENE THERAPY, XX, XX, Bd. 11, Nr. 1, 1. Januar 2000 (2000-01-01), Seiten 9-19, XP000944357 ISSN: 1043-0342
- WATANABE S ET AL: "EXPRESSION OF THE GERM CELL ALKALINE PHOSPHATASE GENE IN HUMAN CHORIOCARCINOMA CELLS" JOURNAL OF BIOLOGICAL CHEMISTRY, AMERICAN SOCIETY OF BIOLOGICAL CHEMISTS, BALTIMORE, MD, US, Bd. 264, Nr. 21, 25. Juli 1989 (1989-07-25), Seiten 12611-12619, XP002225508 ISSN: 0021-9258
- CHRYSOGELOS S A ET AL: "EGF RECEPTOR EXPRESSION, REGULATION, AND FUNCTION IN BREAST CANCER" BREAST CANCER RESEARCH AND TREATMENT, NIJHOFF, BOSTON, US, Bd. 29, Nr. 1, 1994, Seiten 29-40, XP001118068 ISSN: 0167-6806
- FUJIWARA Y ET AL: "ASSESSMENT OF STANNIOCALCIN-1 MRNA AS A MOLECULAR MARKER FOR MICROMETASTASES OF VARIOUS HUMAN CANCERS" INTERNATIONAL JOURNAL OF ONCOLOGY, EDITORIAL ACADEMY OF THE INTERNATIONAL JOURNAL OF ONCOLOGY,, GR, Bd. 16, April 2000 (2000-04), Seiten 799-804, XP002938551 ISSN: 1019-6439
- CHARPENTIER A H ET AL: "STC2 AND E2IG1, TWO NOVEL BREAST CANCER BIOMARKERS" PROCEEDINGS OF THE ANNUAL MEETING OF THE AMERICAN ASSOCIATION FOR CANCER RESEARCH, NEW YORK, NY, US, Bd. 42, Nr. 628, März 2001 (2001-03), Seite 628 XP001119829 ISSN: 0197-016X

## Beschreibung

Die vorliegende Erfindung betrifft ein Verfahren zum qualitativen und/oder quantitativen Nachweis von Zellen in einer Probe. Derartige Nachweisverfahren werden insbesondere bei der Diagnostik oder Behandlungskontrolle von Tumorerkrankungen benötigt. Denn im Rahmen der Krebsvor- oder -nachsorge ist es von großer Wichtigkeit maligne Tumore bzw. rezidive maligne Tumore anhand des Auftretens metastasierender Tumorzellen im Blut frühzeitig nachweisen zu können. Das vorliegende Verfahren wird jedoch nicht nur hier eingesetzt sondern kann ganz grundsätzlich zum Nachweis und zur Erkennung von seltenen Zellen in biologische Zellen enthaltenden Proben eingesetzt werden. Dies kann beispielsweise auch zum Nachweis von fötalen Zellen in maternalem Blut oder auch zum Nachweis von Stammzellen erfolgen.

Hodenkrebs ist für weniger als 2% aller bösartigen Neubildungen von Tumoren beim Mann verantwortlich. Allerdings handelt es sich bei 20-30% aller Krebserkrankungen unter 40jähriger Männer um Hodenkrebs. Die Zahl der jährlichen Neuerkrankungen beispielsweise in der Bundesrepublik Deutschland beträgt ca. 3600, wobei ca. 240 Männer an Hodenkrebs sterben. Die höchste Inzidenz findet man dabei zwischen dem 25. und 40. Lebensjahr. Durch den Fortschritt in der onkologischen Therapie können heute über 90% aller Betroffenen langfristig geheilt werden. Die hohen Überlebensraten begründen sich dabei in der ausgeprägten Wirksamkeit der cis-Platin-basierenden Chemotherapien.

Brustkrebs ist die häufigste Diagnose, wenn eine Tumorerkrankung bei Frauen festgestellt wird (26,4% aller Neuerkrankungen). Trotz massiver Bemühungen, die in Früherkennung, Behandlung und Nachsorge aufgewendet werden, rangiert diese Erkrankung immer noch an erster Stelle krebsbedingter Todesursachen bei der Frau. Die Erkrankungszahlen in den westlichen Industrieländern nehmen in den vergangenen Jahren trotz verstärkter Bemühungen um die Früherkennung weiter zu. Problematisch ist die hohe Metastasierungsrate nach Erstbehandlung, die in der Mehrzahl der Fälle bereits nach 1-3 Jahren zum Tod der Patientin führt. Hauptgrund hierfür ist die Streuung von Tumorzellen in frühen Stadien der Tumorentwicklung. Neben der Ersterkennung eines Mammakarzinoms ist daher insbesondere der frühestmögliche Nachweis metastasierender Zellen für eine erfolgreiche Behandlung von entscheidender Bedeutung. Ebenso kann im klinischen Stadium I ein definitiver Negativnachweis hilfreich sein, wenn zu entscheiden ist, ob die Patientin mit einer Chemotherapie oder einer Operation belastet werden muss.

Beim kolorektalen Primärtumor ist die Tumorprogression nach der Resektion in erster Linie auf residuale Tumorzellen zurückzuführen. Diese Zellen werden prä- oder intraoperativ aus dem primären Tumor abgelöst und erhalten die Möglichkeit zur Verstreuung im ganzen Organismus.

Neben der Ersterkennung eines kolorektalen Karzinoms ist daher insbesondere der frühestmögliche Nachweis metastasierender Zellen für eine erfolgreiche Behandlung von entscheidender Bedeutung.

Dabei gilt es, im klinischen Stadium 1 der Erkrankungen zu entscheiden, ob der Patient mit einer Chemotherapie und/oder mit einer Operation belastet werden muss, um einen dauerhaften Heilungserfolg zu erzielen. Eine große Anzahl von Patienten wird dabei chemotherapeutisch behandelt, obwohl kein gesicherter Nachweis einer Metastasierung vorliegt. In den Konzepten, die auf einer reinen Überwachung aufbauen, kommt es jedoch in 25% der Fälle zu Rezidiven mit zum Teil tödlichem Ausgang.

Bei den derzeit angewandten Untersuchungsmethoden werden bei Krebspatienten sogenannte Tumormarker auf Proteinebene (immunologisch bzw. enzymatisch) quantitativ im Blut oder in anderen Kbrperflüssigkeiten ermittelt. Diese Nachweisverfahren sind jedoch für die Tumordiagnostik bzw. Behandlungskontrolle/Nachsorge bei Tumoren nur bedingt geeignet, da erhöhte Tumormarkerwerte in Körperflüssigkeiten auch durch nichttumoröse Erkrankungen, wie beispielsweise Entzündungen des Magen-Darm-Traktes, Leberzirrhose, Virusinfekte oder starkes Rauchen hervorgerufen werden können.

Molekulargenetische Verfahren erscheinen hier für den Nachweis von Tumorzellen im peripheren Blut hilfreich, da am Beginn des Metastasierungsprozesses der Übertritt von Tumorzellen ins venöse Blut stehen kann.

Die EP 0 520 794 B1 offenbart ein derartiges Verfahren, bei dem Metastasierungen in Körpergeweben oder Flüssigkeiten erfasst werden. Dabei werden Nukleinsäuren erfasst, beispielsweise mittels Vervielfältigung durch Polymerase-Kettenreaktion. Das Verfahren beruht nun entscheidend darauf, dass die nachgewiesene Nukleinsäuresequenz in Zellen des Herkunftsgewebes eines Tumors exprimiert wird, d.h. in Tumorzellen und markerabhängig auch in den gesunden Zellen des Herkunftsgewebes. Weitere Bedingung ist, dass diese Sequenz normalerweise nicht in denjenigen Zellen exprimiert wird, deren Gewebe untersucht wird. Wird also eine entsprechende Sequenz in der untersuchten Probe gefunden, so muss diese von verschleppten, d.h. metastasierenden Zellen eines ortsfremden Tumors herrühren. Damit beruht dieses Verfahren letztlich auf dem Nachweis von Zellen, die in der Blutprobe von gesunden Personen nicht vorkommen sollten.

Insgesamt ist festzustellen, dass die derzeit verwendeten diagnostischen Methoden zu ungenau sind, wenn es um die Beurteilung der malignen Potenz von Resttumoren nach durchgeführter Chemotherapie in den metastasierenden Stadien geht. Es gilt also weiterhin, Nachweise für eine okkulte bzw. restliche Metastasierung zu finden, die eine rechtzeitige Einordnung in die vielfältigen primär kurativen therapeutischen Optionen zulassen. Wesentliches Problem ist es hierbei, dass die zu erfassenden Zellen, beispielsweise Tumorzellen im peripheren Blut, nur in extrem geringer Zahl vorkommen.

Die WO 98/12227 A1 offenbart ein Verfahren zum Nachweis der Expression des GP-54-Antigens. Hierzu werden zuerst aus einer Probe Zellen immunologisch mittels zweier gegen verschiedene Epitope des GP-54-Antigens gerichtete Antikörper isoliert. Für diese isolierten Zellen wird anschließend der Nachweis der Expression von GP-54 durch Analyse der mRNA erbracht.

Die WO 97/37226 A1 offenbart ein Verfahren zur Gewinnung von Tumorzellen oder Fragmenten von Tumorzellen mittels eines Antikörpers bzw. eines Antikörpergemisches. Die Analyse der so gewonnen Zellen oder Zellfragmente erfolgt über optische Verfahren, die es dem Betrachter ermöglichen, Tumorzellen von normalen Zellen zu unterscheiden.

Die WO 96/29430 A1 offenbart die Erfassung von Tumorzellen mit einem Multimarkerassay. Hierbei werden zwei oder mehrere mRNA-Marker in Proben, beispielsweise Blutproben, untersucht. Die Resultate dieses molekularbiologischen Multimarker-Nachweises werden mit einer statistisch signifikanten Referenzgruppe von Normalpatienten und Melanom- oder Brustkrebspatienten verglichen und auf Basis dieses Vergleichs eine Korrelation der Anzahl und Art der Marker mit verschiedenen klinischen Zuständen durchgeführt. Ziel dieses Verfahrens ist es, okkulte Tumoren auch bei Patienten zu erfassen, bei denen keine klinischen Anzeichen für einen Tumor vorhanden sind.

Hardingham et al.: "Immunobead-PCR: A technique for the detection of circulating tumor cells using immunomagnetic beads and the polymerase chain reaction", Cancer Research, Band 53 (1993), Seiten 3455 bis 3458 offenbart ein Verfahren zur Erfassung von disseminierten Tumorzellen in Blutproben. Hierzu erfolgt zuerst ein immunologischer Anreicherungsschritt mittels eines Antikörpers gefolgt von dem Nachweis einer DNA-Mutation. Für den immunologischen Anreicherungsschritt wird aus einer Auswahl von Antikörpern experimentell der geeigneteste Antikörper ermittelt und anschließend für das Verfahren eingesetzt.

Im Kenntnis von diesem Stand der Technik ist es Aufgabe der vorliegenden Erfindung, ein Verfahren zur Verfügung zu stellen, mit dem auf einfache, sichere und wiederholbare Weise seltene biologische Zellen in einer biologische Zellen enthaltenden Probe mit hoher Sensitivität und Spezifität nachgewiesen werden können.

Diese Aufgabe wird durch das Verfahren nach Anspruch 1 gelöst. Vorteilhafte Weiterbildungen des erfindungsgemäßen Verfahrens werden in den jeweiligen abhängigen Ansprüchen gegeben.

Entscheidend bei dem erfindungsgemäßen Verfahren ist es nun, dass zuerst die zu selektionierenden bzw. nachzuweisenden Zellen mittels einer Kombination von Antikörpern oder Antikörperderivaten oder einem bispezifischen Antikörper oder Antikörperderivat markiert werden. Dadurch ist es möglich, insbesondere die gesuchten Zellen zu markieren, abzutrennen und damit anzureichern. Dies bedeutet, dass in einem ersten Schritt ein kombinierter immunologischer Nachweis bzw. Selektionierung erfolgt. Unter Antikörperderivat wird in dieser Anmeldung jede Art von verändertem Antikörper oder Antikörperfragment verstanden, das eine Bindungsstelle aufweist, beispielsweise einkettige Antikörper, Antikörperfragmente wie Fab-Fragmente oder rekombinante Antikörper. Im folgenden sind, wenn von "Antikörper" gesprochen wird, immer Antikörper und/oder Antikörperderivate bezeichnet.

In einem zweiten Schritt werden dann auf molekularbiologischer Basis mit einer vordefinierten Kombination von Nachweisreagentien mindestens ein Marker erfasst, der für die gesuchten Zellen spezifisch ist bzw. in diesen präferentiell zu finden ist, so dass hier wiederum spezifisch die gesuchten Zellen ausgewählt werden. Es handelt sich hier also um einen kombinierten molekularbiologischen Nachweis. Grundgedanke der vorliegenden Erfindung ist also, einen Nachweis über eine Kombination immunologischer Parameter mit einem Nachweis über eine Kombination molekularbiologischer Parameter zu kombinieren. Überraschenderweise ergeben sich hierdurch ganz hervorragende Nachweisergebnisse, mit denen in Detektionsbereiche vorgestoßen wird, die sämtlichen bekannten Techniken aus dem Stand der Technik bisher nicht zugänglich waren. So können Konzentrationen gesuchter Zellen in Blutproben bis herab zu zwei Zellen pro 5 Milliliter noch nachgewiesen werden. Eine derartige Spezifität und Sensitivität wird bisher im Stand der Technik nicht erreicht.

Eukaryontische Zellen tragen eine Vielzahl unterschiedlicher Moleküle an ihrer Zelloberfläche. Entsprechend des Ursprungs und der Funktion der einzelnen Zelle unterscheidet sich die Kombination der exprimierten Oberflächenmoleküle, so dass zelltypspezifische Muster entstehen. Zur Erkennung dieser zelltyp-spezifischen Muster werden Antikörper genutzt. Antikörper binden mit hoher Spezifität an ihr Antigen, hier an ausgewählte Oberflächenmoleküle. Diese Eigenschaft wird genutzt, um Zellen mittels spezifischer Antikörperbindung anhand ihrer Zelltyp-spezifischen Muster zu erkennen und voneinander zu unterscheiden.

So unterscheidet beispielsweise die Expression spezieller Oberflächenproteine von Tumorzellen von nichttranaformierten Zellen dieses Zelltyps.

Dem Nachweis der Marker geht eine Selektion der Zielzellen über die Bindung verschiedener Antikörper an die gesuchten Zellen voraus. Denn die Expression spezieller Oberflächenproteine unterscheidet Zellen eines Typs von Zellen eines anderen Typs. So unterscheidet beispielsweise die Expression spezieller Oberflächenproteine Tumorzellen von nicht-transformierten Zellen dieses Zelltyps.

Da sich das spezielle Muster der Oberflächen-Antigene beispielsweise bei Tumorzellen auch von den Blutzelltypischen Mustern unterscheidet, können Tumorzellen im Blut unterschieden werden. Um Tumorzellen zu identifizieren, werden Antikörper, die diese speziellen Oberflächenproteine spezifisch erkennen, als Werkzeuge genutzt. Die spezifische Antikörperbindunq wird für verschiedene Analyse- und Separations-Methoden nutzbar gemacht.

Aufgrund der intensiven Bindung von speziell dafür selektionierten Immunglobulinen ist neben der Erkennung von Zellen über deren Oberflächenepitope auch eine Separierung der erkannten Zellen von nicht erkannten möglich.

Verschiedene Trennprinzipien sind möglich:
1. Trennprinzip beruhend auf Flüssigphase; z.B. Durchflusszytometrie:
   Für die durchflußzytometrische Analyse werden Antikörper mit Fluoreszenzfarbstoffen gekoppelt. Vereinzelte Zellen werden in einem konstanten Flüssigkeitsstrom einzeln an einer Lichtquelle (Laser) vorbeigeleitet. Bei Beleuchtung der Zellen werden die an den Antikörpern gebundenen Fluoreszenzfarbstoffe angeregt und strahlen Licht bestimmter Wellenlängen ab. Das abgestrahlte Licht wird detektiert und das gemessene Signal digitalisiert gespeichert. Das Lichtsignal kann einzelnen Zellen zugeordnet werden. Die Antikörper-markierte Zelle wird so erkannt und kann nun von anderen Zellen getrennt werden. Zur Trennung werden die Zellen in kleinsten Tropfen vereinzelt. Nach Erkennung der Antikörper-markierten Zelle wird der entsprechende Tropfen in ein Auffangbehältnis gelenkt. Eine derartige Anreicherung kann beispielsweise durch FACS-Durchflusszytometrie erfolgen. Dabei werden beispielsweise angereicherte Zellen mit fluoreszenzmarkierten monoklonaren Antikörpern gegen tumorspezifische Oberflächenproteine inkubiert. Die markierten Zellen werden zweifach mit PBS gewaschen und im Anschluss werden 10⁷ Zellen in 1 ml PBS resuspendiert. Für die Isolierung der Tumorzellen wird ein FACS Vantage SE-Durchflusszytometer (Becton Dickinson) verwendet. Über das CellQuest Programm erfolgen Datenaufnahme, Instrumentenkontrolle und Datenauswertung. Die sortierten Zellen werden in ein 1,5 ml-Reaktionsgefäß (gefüllt mit 1 ml PBS) überführt. Die RNS kann dann wie später beschrieben isoliert werden.
2. Trennprinzip beruhend auf Festphase; z.B. magnetische Separation:
   Für die magnetische Separation werden Antikörper an pseudomagnetische Partikel gekoppelt. Nach Einbringen der pseudomagnetischen Partikel in ein Magnetfeld wandern die Partikel im magnetischen Feld. Bei der Bewegung in diesem magnetischen Feld werden Zellen, an die diese gekoppelten Antikörper gebunden sind, mitgerissen und von anderen Zellen getrennt.

Zur Zellerkennung mittels Magnetpartikel werden folglich an pseudomagnetische Partikel, die eine definierte Anzahl an chemisch aktivierten Stellen auf ihrer Oberfläche besitzen, Antikörper gekoppelt. Kopplungsverfahren sind beispielsweise aus James P. Gosling, Solid-phase Concepts and Design, in: R.F. Masseyeff, W.H. Albert N.A. Staines (eds), Methods of Immunological Analysis, Vol. 1, VCH Verlagsgesellschaft mbH, Weinheim, pp. 507-529 bekannt. Über die Spezifität der Antikörper wird die Spezifität der Trennung bestimmt. Eine Ziel-Zellen enthaltende Blutprobe wird mit Antikörper-gekoppelten Magnetpartikeln versetzt; dann werden Partikel und Blut relativ zueinander bewegt, beispielsweise durch "Über-Kopf-Rotieren" in einem geschlossenen Behälter befindlicher Proben oder durch Bewegung der Partikel aufgrund wechselnder Magnetfelder. Jene Ziel-Zellen, die von einem der Festphase-gebundenen Antikörper erkannt und damit fest gebunden werden, folgen der Bewegung der Partikel. Hierdurch ist es möglich, bei Anlegen eines magnetischen Feldes, die Partikel mit den daran gebundenen Zellen aus dem Blut herauszuziehen (z.B. an die Wand des Trenngefäßes). Das auf diese Weise Ziel-Zellen-depletierte Blut kann gegen andere Lösungen ausgetauscht werden, wobei die über Magnetpartikel separierten Zellen bis zum Abschalten/Entfernen des Magnetfeldes vor Ort verbleiben und für weitere Anwendungen zur Verfügung stehen.

Alternativ zu den dargestellten Trennprinzipien sind auch jegliche andere Trennprinzipien aus dem Stand der Technik, die auf der Markierung von Zellen mit Antikörpern beruhen, einsetzbar.

Erfindungsgemäß werden vorteilhafterweise für die Erkennung der Tumorzellen spezifische AntikörperMischungen verwendet. Beispielsweise eignet sich zur Erkennung von Tumorzellen im Blut eine Kombination der Antikörper MOC-31 und Ber-EP4.

**Tabelle 1:**

| **Antikörper-Mischung** | | |
|---|---|---|
| **Antigen** | **Klon** | **Konzentration** |
| Epith.Rel.Antigen | MOC-31 (Fa. Novocastra) | 1,25 µl/10⁸ Zellen |
| Epitheliales Antigen | Ber-EP 4 (Fa. DAKO) | 0,924 µg/10⁶ Zellen |

Mittels der Antikörpermischung in der vorhergehenden Tabelle 1 werden bevorzugt Tumorzellen, jedoch mit hoher Spezifität erfasst. Dies beruht auf der bevorzugten Expression bestimmter Oberflächenproteine, die Krebszellen von anderen Zellen unterscheidet.

Derartige Antikörpermischungen zeigen im Vergleich zu den jeweils separat eingesetzten Antikörpern bei der Zellerkennung und Zelltrennung unabhängig von der angewandten Methode eine erhöhte Sensitivität.

Die vorliegende Erfindung beruht weiterhin wesentlich darauf, dass nicht etwa auf immunologischer oder enzymatischer Ebene Zellmarker im Blut von Patienten nachgewiesen werden, sondern dass als molekularbiologischer Marker die mRNS (Messenger-Ribonukleinsäure) von gesuchten Zellen in einer Probe beispielsweise einer Blutprobe, nachgewiesen wird.

Da einzelne Marker in therapieabhängiger Weise unterschiedlich exprimiert werden, wird eine Kombination von Tumormarkern untersucht, um alle im Blut zirkulierenden Tumorzellen zu erfassen. Hierdurch lassen sich Tumorzellen auch dann erkennnen, wenn die Expression eines bestimmten Markers bei einem Patienten bzw. in einem Krankheitsstadium relativ gering ist, was sonst zu einem vermeintlich negativen Ergebnis führen könnte. Die Verwendung von Markern stößt jedoch meist deswegen auf Grenzen, weil mononukleäre Blutzellen eine Hintergrundexpression ("illegitime Transkription") aufweisen, die eine exakte Analyse behindern.

Als Marker, beispielsweise für Tumoren, wird die Expression der in Tabelle 2 genannten Gene erfasst. Der Nachweis kann dabei für zwei Marker oder auch für eine beliebige Anzahl dieser Tumormarker in Kombination miteinander durchgeführt werden. Das erfindungsgemäße Kit kann daher Oligonukleotidpaare für zwei oder eine beliebige Auswahl oder auch alle der Tumormarker enthalten.

**Tabelle 2**

| **Gen bzw. Genprodukt** | **Gen** | **Alernativ- bezeichn..** |
|---|---|---|
| Human carninoma-associated antigen GA733-2 gene | GA733-2 | GA733.2 |
| Human epidermal growth factor receptor (EGFR) gene | EGFR | EGFR |
| Human carcinoembryonic antigen (CEA) gene | CEA | CEA |
| Homo sapiens mucin 1 (MUC1) | MUC1 | CA15-3 |
| Homo sapiens C-erb B2/neu protein (ERBB2) gene | HER-2/neu | HER-2 |
| Homo sapiens claudin 7 (CLDN7), mRNA | claudin7 (CLDN7) | Claudin-7 |
| Alkaline phosphatase, placental-like (Nago isozyme), (Germ-cell alkaline phosphatase), (PLAP-like) | ALPPL2 (GCAP) | PLAP |
| Homo sapiens gastrin-releasing peptide receptor (GPPR) gene | GRPR | GRPR |
| Homo sapiens high-mobility group (nonhistone chromosomal) protein isoform I-C (HMGIC), mRNA | HMGIC | HMGI-C |
| Homo sapiens gene for cytokeratin 20 | CK20 | CK20 |
| Human MAGE-3 antigen (MAGE-3) gene | MAGE-3 | MAGE-3 |
| Homo sapiens stanniocalcin 1 (STC1) gene | Stanniocalcin 1 (STC1) | Stanniocalcin |

Dadurch werden alle diejenigen Fälle richtigerweise vernachlässigt, bei denen beispielsweise aufgrund von anderen Krankheiten ebenfalls die Tumormarker exprimiert werden und lediglich als Protein in die Blutbahn gelangen. Es werden folglich aufgrund des ersten immunologischen Auswahlschrittes lediglich Zellen erfasst, die zum einen sich selbst in der Blutprobe befinden und zum anderen den oder die jeweiligen Tumormarker exprimieren. Dabei handelt es sich folglich um Tumorzellen, die aus ihrem ursprünglichen Tumorgewebe stammen und in das Blut der Patienten verschleppt wurden. Da im Blut nicht an einem Tumor Erkrankter die mRNA der untersuchten Marker normalerweise nicht exprimiert wird, zeigt sich eine direkte Korrelation zwischen dem Auftreten der zugehörigen mRNA und einer Metastasierung schon im frühen Stadium im Metastasierungsprozess.

Dabei wird nicht nur die mRNS eines einzelnen Tumormarkers erfasst sondern eine Kombination von Markern untersucht. Dadurch ist es möglich, Krebsformen über ihre im Blut metastasierenden Zellen erfassen zu können. Dies bedeutet, dass im Falle von Hodentumoren sowohl seminöse als auch nichtseminöse Hodenkrebsformen bzw. auch Mischtumore mit Anteilen eines Seminoms und damit 90-95 % aller malignen Tumore des Hodens, nämlich sämtliche Keimzelltumoren, erfasst werden.

Für die Erkennung von Hodentumorzellen wird daher erfindungsgemäß eine Kombination von mindestens zwei der folgenden Marker vorgeschlagen:
- GA733.2
- GCAP/PLAP
- HMGI-C
- GRPR,

Für die Erkennung von Brustkrebszellen wird erfindungsgemäß eine Kombination von mindestens zwei Tumormarkern der folgenden Markergruppen vorgeschlagen:
a) EGFR, CEA, Stanniocalcin, MAGE 3, CK20, Claudin 7, Her-2/neu, MUC1 und GA 733.2;
b) CK20, MAGE 3 und MUC1
c) Her-2/neu und Claudin7 sowie
d) EGFR, CEA und Stanniocalcin

Für die Erkennung von Darmkrebszellen wird erfindungsgemäß eine Kombination von mindestens zwei Tumormarkern der folgenden Markergruppen vorgeschlagen:
a) CK20, EGFR, GA 733.2, CEA und Stanniocalcin
b) CK20, EGFR, CEA und Stanniocalcin sowie
c) EGFR, CEA und GA 733.2

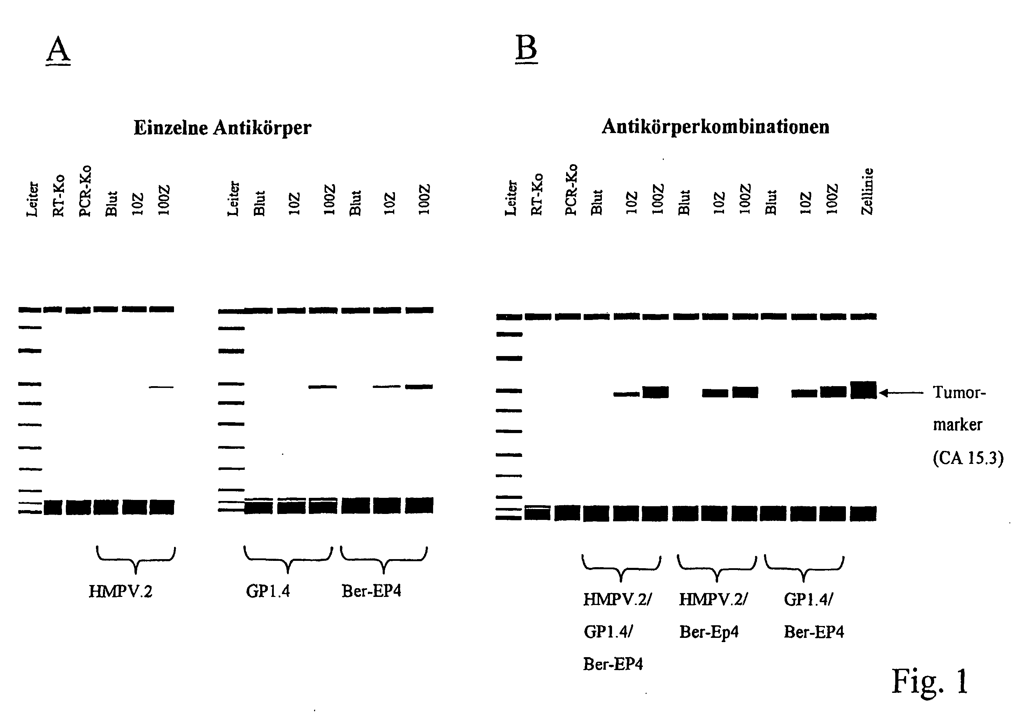

Im folgenden werden einige Beispiele gegeben, aus denen hervorgeht, daß mit dem erfindungsgemäßen Verfahren eine Nachweissensitivität erzielt wird, die über alles bisher im Stand der Technik bekannte weit hinausgeht. Die Figuren 1 bis 10 zeigen Ergebnisse verschiedener Versuchsprotokolle.

Gemeinsam für sämtliche Beispiele ist das grundsätzliche Vorgehen, das einen ersten Schritt mit immunologischer Anreicherung von Zielzellen und einen zweiten Schritt eines Nachweises von mRNS-Markern in den immunologisch angereicherten Zellen umfaßt. Im folgenden werden diese Schritte in allgemeiner Form beschrieben, soweit sie für sämtliche Beispiele identisch sind.

### 1. Immunologische Anreicherung der Zielzellen aus peripherem Blut

Zuerst wurde eine periphere Blutprobe entnommen und dieser eine definierte Anzahl von Zielzellen hinzugegeben, beispielsweise 2, 10, 100 Zellen eines bestimmten Tumortyps.

Weiterhin wurden Antikörper an Magnetpartikel gekoppelt. Als Antikörper wurden dabei die im folgenden in Tabelle 3 dargestellten Antikörper verwendet.

**Tabelle 3**

| Antigen | Klon | Firma |
|---|---|---|
| Epitheliales Membran Antigen | GP1.4 | Novocastra |
| Epitheliales Antigen | MOC-31 | Novocastra |
| Epitheliales Antigen | Ber-EP4 | DAKO |
| Muc 1 | HMPV.2 | Pharmingen |
| PLAP | 8B6 | Cymbus Biotechnology LTD |
| Epitheliales Membran Antigen | E29 | DAKO |
| Epitheliales Membran Antigen | 131-11741 | HISS |

Die Magnetpartikel wurden dabei mit einer Partikelkonzentration von 4 x 10⁸ beads/ml (CELLection™ Pan Mouse IgG Kit, Firma Dynal) verwendet. Die Verhältnisse zwischen der Antikörperkonzentration und den daran gekoppelten Antikörpern sind in Tabelle 4 wiedergegeben.

**Tabelle 4**

| Klon | Antikörperkonzentration | µl Antikörper/25 µl Partikel |
|---|---|---|
| BerEP4 | 0,1 mg/ml | 4 µl |
| HMPV.2 | 0,5 mg/ml | 4 µl |
| MOC31 | k.A. Verdünnung s. Herstellerangaben (Lyophilisat) | 4 µl |
| GP1.4 | k.A. Verdünnung s. Herstellerangaben (Lyophilisat) | 4 µl |
| 8B6 | 0,1 mg/ml | 1 µl |
| 131-11741 | 0,5 mg/ml | 4 µl |
| E29 | 0,1 mg/ml | 4 µl |

Die so vorbereiteten Magnetpartikel wurden je nach Versuchsansatz und Nachweissystem dem Blut zugegeben. Der entsprechende Zusatz Antikörper-gekoppelter Magnetpartikel pro ml Blut bei einer Ausgangskonzentration von 4 x 10⁸ beads/ml Partikel ist in Tabelle 5 wiedergegeben.

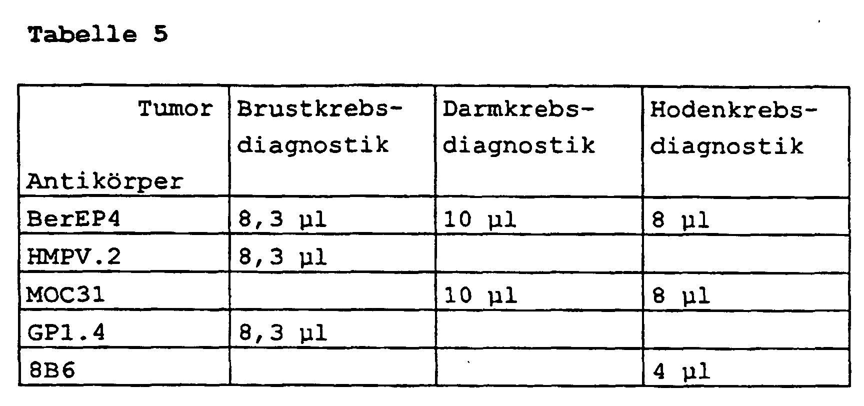

Nach 2-stündiger Inkubation im Überkopfschüttler wurden die Magnetpartikel, die gegebenenfalls als Zell-Antikörper-Magnetpartikelkomplexe vorlagen, mittels eines Magnetpartikelkonzentrators (MPC®-S, Firma Dynal) 3 mal mit PBS (Phosphatpuffer-Saline) gewaschen und die anhaftenden Zellen anschließend entsprechend des im folgenden beschriebenen RNS-Isolierungsprotokolls behandelt.

Als Alternative zur Abtrennung mittels Magnetpartikeln bietet sich eine immunologische Abtrennung mittels Durchflußzytometrie (Fluoreszenz-assoziierte Zellsortierung, FACS) an.

Hier wird eine erste relative Anreicherung der Tumorzellen durch Depletion der Erythrozyten erzielt. Dazu wird Vollblut (mit EDTA) mit einem hypotonen Erythrozyten-Lyse-Puffer vermischt und 30 Minuten bei Raumtemperatur inkubiert. Die verbliebenen kernhaltigen Zellen werden zentrifugiert und in PBS/BSA resuspendiert. Die so gewonnenen Zellen werden anschließend mit Antikörpern inkubiert, die mit einem Fluorophor markiert sind. Die Zielzellen, die durch Bindung an einen Antikörper fluoreszierend markiert sind, wurden dann über FACS abgetrennt.

Alternativ bietet sich eine Anreicherung durch Dichtegradienten-Zentrifugation an. Durch eine derartige Zentrifugation mit unterschiedlichen Dichtegradienten werden Zellen unterschiedlicher mittlerer Volumendichte voneinander getrennt. Mononukleare Blutzellen werden mittels eines Ficoll-Hypaque-Gradienten (Firma Pharmacia, Uppsala, Schweden) separiert und anschließend zweifach mit PBS/1% FCS gewaschen. Anschließend erfolgt eine Festphasen-gekoppelte Anreicherung (z.B. über Magnetpartikel) bzw. eine Flüssigphasen-basierte Abtrennung (FACS) der Zielzellen wie oben beschrieben.

### 2. mRNS-Isolierung

Als erstes erfolgt eine Isolierung der Gesamt-RNS der wie oben beschrieben abgetrennten Zellen. Diese erfolgt mit dem QIAamp RNA Blood Mini Kit (Firma Qiagen, Hilden) nach dortigen Herstellerangaben, wobei der Lysis-Puffer direkt zu den an die Magnetpartikel gebundenen Zellen gegeben wurde. Durch einen zusätzlichen DNS-Verdau auf der Säule wird eine Kontamination mit genomischer DNS vermieden. Dieser DNS-Verdau erfolgt mit dem RNase-Free DNase Set, Firma Qiagen, Hilden.

Alternativ kann auch eine mRNS-Isolierung, z. B. mittels Oligo(dT)-gekoppelter Magnetpartikel, Dynabeads® mRNA Direct™ Micro Kit, (Firma Dynal)) erfolgen. Auch diese Isolation erfolgt entsprechend der in dem Kit angegebenen Herstellerangaben.

Als weitere Alternative zur RNS-Isolierung werden die isolierten Zellen durch Zugabe von Trizol-Reagenz (Firma Gibco BRL, NY, USA) lysiert und mittels Pipette homogenisiert. Nach anschließender Chloroformextraktion wird die RNS-haltige wäßrige Phase in Isopropanol bei -80 °C gefällt. Nach zweimaligem Waschen und Zentrifugieren in 80 %igem Ethanol wird das Pellet an der Luft getrocknet und anschließend in RNase freiem Wasser resuspendiert. Dieser Aufarbeitungsschritt erfolgt ebenfalls nach herkömmlichen Protokollen.

### 3. Reverse Transkription

An die Isolierung der RNS schließt sich eine reverse Transkription an, bei der die mRNS in cDNS umgeschrieben wird.

Dazu wird die RNS in einem entsprechenden Volumen Wasser gemäß dem Reaktionsansatz in Tabelle 6 zusammen mit Oligo(dT)15-Primern (Firma Promega, Mannheim) für 5 Minuten bei 65 °C denaturiert und anschließend direkt auf Eis inkubiert.

**Tabelle 6:**

| **Komponenten der cDNS-Synthese** Die cDNS-Synthese erfolgt in einem 20 µl-Reaktionsansatz | | |
|---|---|---|
| Komponenten | Volumen | Endkonzentration |
| RNS/mRNS | 10 µl | - |
| 10 x RT-Puffer | 2 µl | 1 x |
| dNTP-Mix (je 5 mM) | 2 µl | jeweils 0.5 mM |
| Oligo(dT)-Primer (10 µM) | 2 µl | 1 µM |
| RNase-Inhibitor | 1 µl | 0.5 Units/µl |
| Reverse Transkriptase | 1 µl | 4 U |
| RNase-freies Wasser | ad 20 µl | |

Die cDNS-Synthese erfolgte bei 37 °C für eine Stunde mit nachfolgender Inaktivierung der reversen Transkriptase durch Erhitzen für 5 Minuten bei 95 °C und anschließender Abkühlung auf Eis. Hierzu wurde ein Sensiskript Reverse Transkriptase Kit, Firma Qiagen, Hilden nach dort angegebenen Protokollen verwendet.

Im Falle der Verwendung Oligo(dT)-gekoppelter Magnetpartikel bereits zur Isolierung von mRNS unterbleibt anschließend die Zugabe von Oligo(dT)-Primern, d.h. der Oligo(dT)-Linker dient zugleich als Primer für die reverse Transkription, wobei hier die Magnetpartikel im Ansatz verbleiben.

### 4. PCR

Anschließend an die Umschreibung der mRNS in cDNS erfolgt eine Polymerase-Kettenreaktion (PCR) mit β-Aktin als interner Kontrolle.

Dabei wurden die in Tabelle 7 aufgeführten Oligonukleotide als PCR-Primer zur Amplifikation von cDNS entsprechend verschiedener Markergene, wie sie in der ersten Spalte angegeben sind, verwendet.

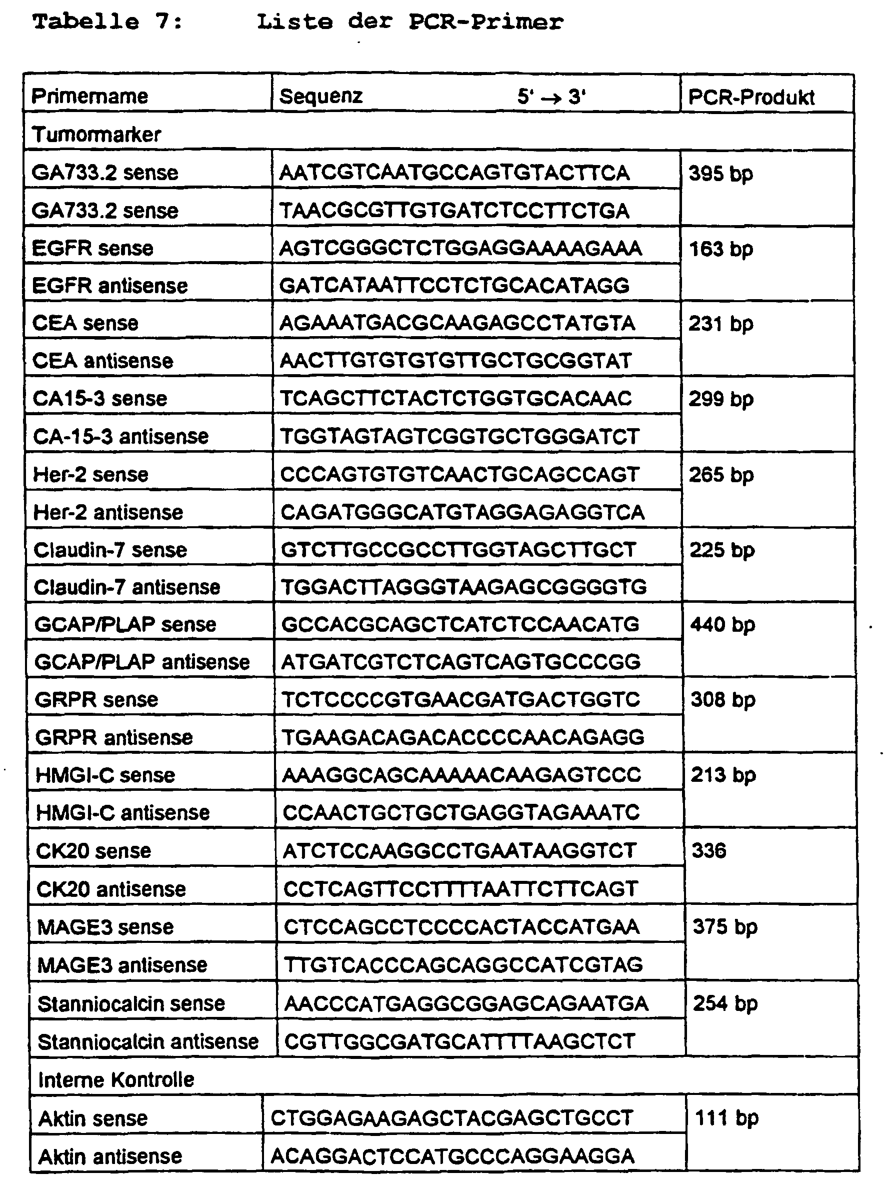

Tabelle 7 enthält in der ersten Spalte die Angabe des zu erfassenden Tumormarkers, wobei jeweils zwei Oligonukleotide (sense und antisense) als Primerpaar angegeben sind. Die Länge des PCR-Produktes, das durch die in Spalte zwei angegebenen Primer erzeugt wird, ist in Spalte drei angegeben. Die PCR wurde mit dem in Tabelle 8 angegebenen Ansatz durchgeführt.

**Tabelle 8:**

| **PCR-Ansatz** Die PCR-Synthese erfolgte in einem 50 µl-Rekationsansatz | | |
|---|---|---|
| Komponenten | Volumen | Endkonzentration |
| cDNS | 6 µl | |
| 10 x PCR-Puffer* | 5 µl | 1 x |
| dNTP-Mix | 1 µl | jeweils 200 µM |
| Primer | s. Tabellen 7 und 9 | |
| Taq-DNA Polymerase** | 0,5 µl | 2.5 U |
| [Q-Solution*** | 10 µl] | |
| H₂O | ad 50 µl | |

| | | |
|---|---|---|
| (* enthält 15 mM MgCl₂; ** HotStarTaq™ DANN Polymerase; Qiagen, Hitden) | | |
| (*** Der Zusatz von 10 µl Q-Solution (Qiagen, Hilden) ist nur zum Nachweis von GCAPIPLAP notwendig) | | |

Tabelle 9 gibt eine Auflistung der spezifischen Primerkombination und Primerkonzentrationen als Endkonzentration im PCR-Ansatz an. In den folgenden Beispielen wird für die verschiedenen Tumorarten Brustkrebs, Darmkrebs und Hodenkrebs jeweils exemplarisch eine Multiplexkombination zu diesen Primern aufgezeigt, so wie sie in Tabelle 9 angegeben ist.

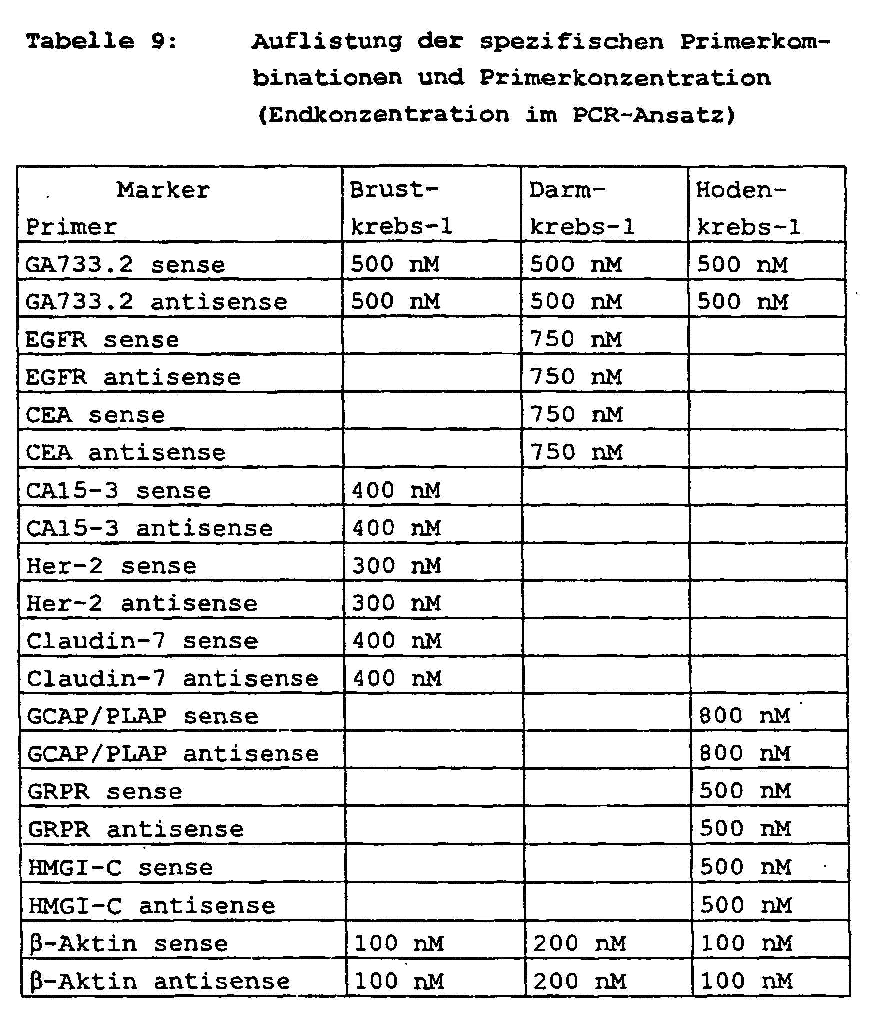

Die PCR-Bedingungen (Zyklenzahl, Zyklenführung etc.) sind in den Tabellen 10 und 11 gegeben.

**Tabelle 10:**

| PCR-Bedingungen | | |
|---|---|---|
| Vorabdenaturierung | | 95 °C 15 min |
| Zyklus | | |
| 1. | Denaturierung | 94 °C 1 min |
| 2. | Annealing | x °C 1 min (s. Tabelle 11) |
| 3. | Extension | 72 °C 1 min |
| Finale Extension | | 72 °C 10 min |
| | | 4 °C Pause |

**Tabelle 11:**

| Multiplex-spezifische Annealingtemperatur und Zyklenzahl | | | |
|---|---|---|---|
| Marker | Brustkrebs-1 | Darmkrebs-1 | Hodenkrebs-1 |
| Annealing-Temperatur | 60 °C | 58 °C | 58 °C |
| Zyklenzahl | 35 | 40 | 40 |
| (Thermocycler: PCT 200; Biozym) | | | |

Die so erzeugten Amplifikate der cDNS wurden elektrophoretisch aufgetrennt mittels eines Bioanalyzer 2100 (Firma Agilent). Hierzu wurde 1 µl des PCR-Produktes in dem Bioanalyzer auf einem DNS-Chip (500) aufgetrennt und das Trennergebnis elektronisch dokumentiert. Auf diese Weise wurden die Figuren 1-10 erzeugt.

Alternativ werden 25 µl des PCR-Produktes über ein 2,5 %iges Agarosegel aufgetrennt und die DNS-Banden mit Ethidiumbromid angefärbt. Die Dokumentation erfolgt z.B. mit Hilfe des DUO Store Systems der Firma Intas.

Alternativ kann weiterhin eine Fragmentanalyse beispielsweise mittels eines ABI Prism 310 Genetic Analyser (Firma PE Applied Biosystem, Weiterstadt) durchgeführt werden. Hierzu wird dann jeweils 1 µl des PCR-Produktes in der Verdünnung 1:50 eingesetzt. In diesem Falle werden fluoreszenzmarkierte Primer verwendet.

Figur 1 zeigt nun das Ergebnis eines erfindungsgemäßen Verfahrens für die Erkennung von Brustkrebszellen im Blut. Hierzu wurde Blut von Gesunden eine definierte Menge an Tumorzellen einer Brustkrebszellinie zugegeben. Die zugegebene Zellzahl betrug dabei 10 Zellen (10 Z)bzw. 100 Zellen (100 Z)pro Milliliter Blut. Figur 1A und 1B zeigen nun jeweils elektrophoretische Auftrennungen, wobei die einzelnen Banden hier und im folgenden mit denselben Begriffen beschriftet sind. Der Begriff Leiter bezeichnet Calibratoren von 50-600bp Länge, mit RT-Ko ist eine Kontrolle bezeichnet, die keinerlei mRNA enthielt, mit PCR-Ko ist eine Kontrollmessung bezeichnet, die keinerlei cDNS vor der PCR enthielt. Mit "Blut" ist die Blutprobe ohne inokulierte Tumorzellen, mit 10 Z die Blutprobe mit 10 inokulierten Tumorzellen pro Milliliter und mit 100 Z die Blutprobe mit 100 inokulierten Tumorzellen pro Milliliter bzw. pro 5 Milliliter bezeichnet. Mit "Zellinie" ist eine Kontrollmessung mit einer großen Zellzahl der Tumorzellinie in der Probe bezeichnet.

In Figur 1 sind Ergebnisse dargestellt, wenn die Selektion im ersten Schritt mit lediglich einem, zweien oder allen drei der folgenden Antikörper HMPV.2, GP1.4 und Ber-Ep 4 durchgeführt wurde. Es ist unmittelbar zu erkennen, daß bei Verwendung lediglich eines Antikörpers der Nachweis des Tumormarkers CA 15.3 (Muc1) nur gering ist. Die besten Ergebnisse werden erzielt, wenn zwei der Antikörper, nämlich HMPV.2 und Ber-Ep 4 bzw. GP1.4 und Ber-Ep 4 zum Nachweis eingesetzt werden. Bereits die Kombination aller drei Antikörper ist, wie man an der Intensität der Banden für den Tumormarker CA 15.3 erkennen kann, effektiver. Damit ist nachgewiesen, daß bei geeigneter Auswahl einer bestimmten Anzahl von spezifischen Antikörpern ein erheblich verbessertes Ergebnis beim Nachweis von Tumorzellen möglich ist. Insbesondere zeigt sich auch, daß der simple Schluß, daß unter Einsatz von mehreren Antikörpern die Sensitivität zwangsweise steigen würde, nicht möglich ist. Das Gegenteil ist unter Umständen der Fall, da eine unspezifische Reaktion mit steigender Zahl von Antikörpern eher möglich wird. Es ist daher von besonderer Bedeutung, experimentell eine geeignete Kombination von Antikörpern zu ermitteln.

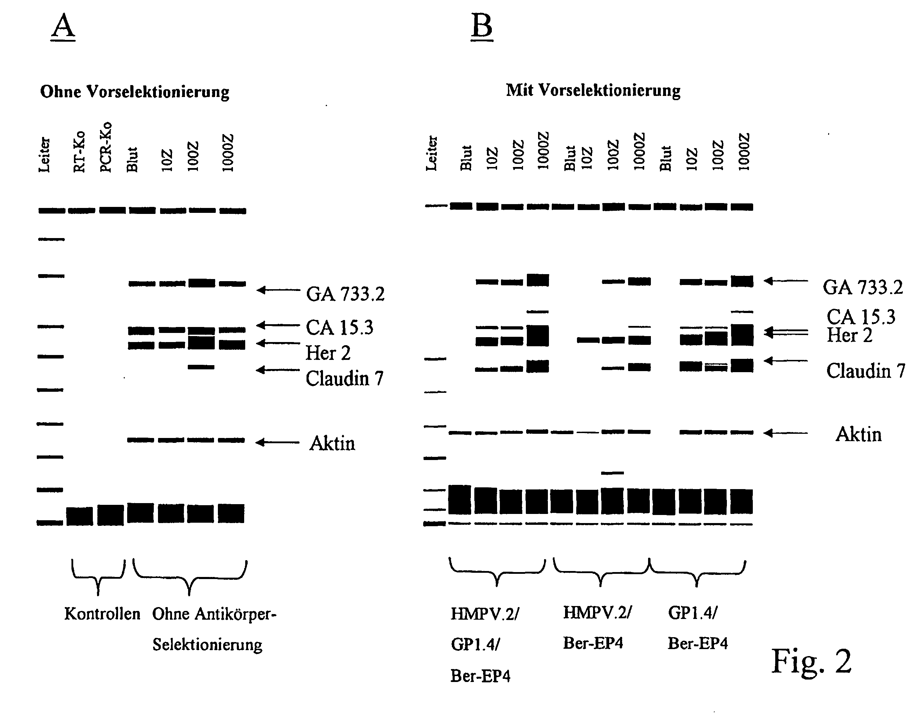

Figur 2 zeigt nun Ergebnisse von Nachweisverfahren, bei denen in Figur 2A keine Vorauswahl mittels Antikörpermarkierung und in Figur 2B eine Vorauswahl mittels Antikörpermarkierung durchgeführt wurde. In Figur 2 sind dabei Kombinationen der Antikörper HMPV.2, Ber-Ep 4 und GP1.4 als Zweierkombination bzw. sämtlicher drei Antikörper bestimmt worden. Zugleich wurde eine Multiplexbestimmung von insgesamt vier Markern, nämlich GA 733.2, CA 15.3, Her 2/neu sowie Claudin 7, durchgeführt. Auch hier wurden wieder 10, 100 bzw. 1000 Tumorzellen einer Brustkrebszellinie in Blut inokkuliert und anschließend nachgewiesen. Hier zeigt sich, daß ohne Antikörper-Selektionierung ein Hintergrundexpression für einige der mRNS-Marker (GA 733.2, CA 15.3 und Her 2/neu) erfaßt wird. Ein derartiger Hintergrund läßt sich bei Einsatz jeder der in Figur 2B dargestellten Antikörperkombinationen zur Vorauswahl der auf mRNS zu untersuchenden Zellen vermeiden. Interessant ist in Figur 2B wiederum, daß der Einsatz von drei Antikörpern dem Einsatz von zwei Antikörpern, z. B. GP 1.4 mit Ber-Ep 4 nicht unbedingt überlegen ist. Die Auswahl bestimmter Antikörperkombinationen sowie die Auswahl bestimmter mRNS-Marker ermöglicht es jedoch, bis herab zu 10 Zellen pro Milliliter Blut ohne jeglichen unspezifischen Hintergrund die entsprechenden gesuchten Tumorzellen zu erfassen. Die Hintergrundexpression konnte eliminiert und die Sensitivität beträchtlich gesteigert werden (s. Bande für Claudin 7).

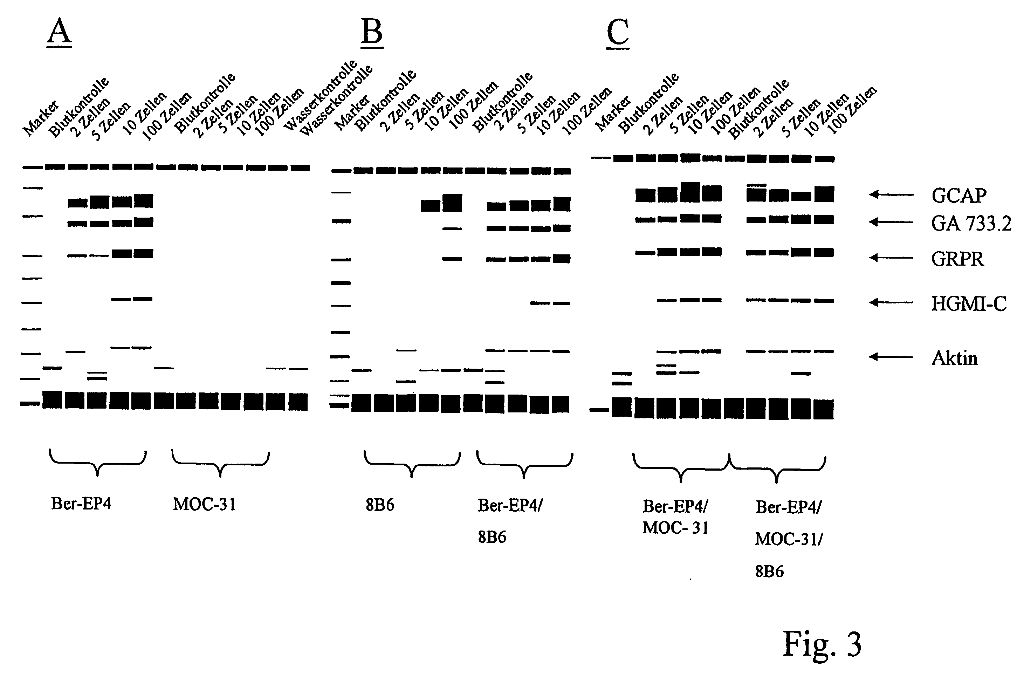

In Figur 3 ist das Ergebnis eines erfindungsgemäßen Verfahrens mit in Blut inokulierten Tumorzellen aus einer Hodenkrebszellinie dargestellt. Wiederum werden sämtliche Tumorzellen aus der Blutprobe selektiert, die durch einen der dargestellten Antikörper markiert sind. Anschließend wird auf insgesamt vier mRNS-Marker (GCAP, GA 733.2, GRPR und HGMI-C) untersucht. Hier zeigt sich, daß bei Einsatz nur eines Antikörpers wie Ber-Ep 4 mittels des Markers HGMI-C lediglich bis herab zu 10 Zellen pro Milliliter Blut erfaßt werden und bei Einsatz des Antikörpers MOC-31 überhaupt keine Hodenkrebszellen erfaßt werden. Dasselbe gilt für den Antikörper 8B6, der lediglich eine geringe Sensitivität aufweist.

Die Kombination der Antikörper Ber-Ep 4 und 8B6 führt ebenfalls zu einem mangelhaften Nachweis mittels des Markers HGMI-C sowie auch die Kombination der Antikörper Ber-Ep 4 und MOC-31. Ein optimales Nachweisergebnis für sämtliche vier untersuchten Marker ergibt sich für Hodentumorzellen bei Einsatz von insgesamt drei Antikörpern Ber-Ep 4, MOC-31 und 8B6, wo bis herab zu 2 Zellen pro Milliliter Blut sicher über jeden einzelnen der Marker nachgewiesen werden. Werden erfindungsgemäß mit den zwei Nachweisreaktionen zwei Marker nachgewiesen, so läßt sich bei Auswahl von zwei Markern aus den für Figur 3 verwendeten Markern ein sicherer Nachweis einer minimalen Zellenzahl bei gleichzeitiger Vermeidung einer Hintergrunderfassung durchführen.

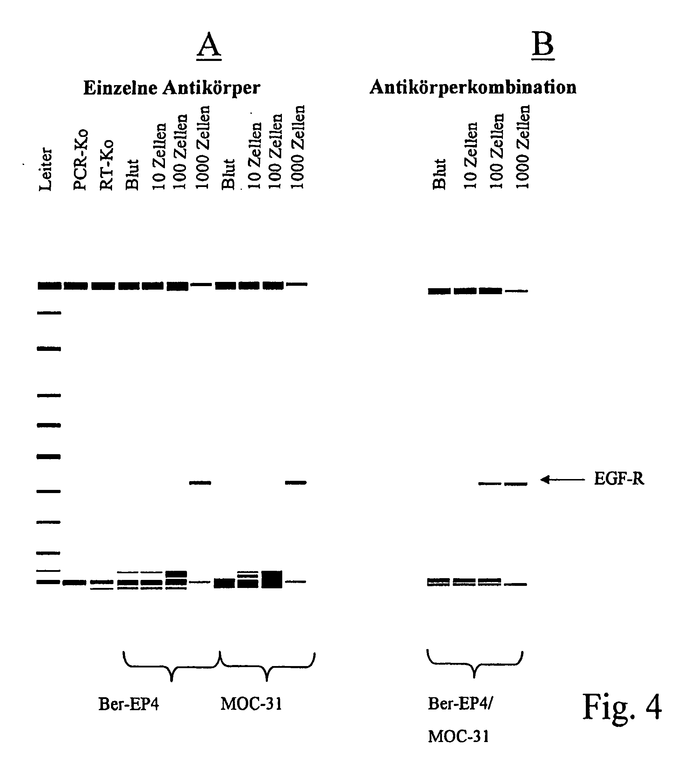

Figur 4 zeigt den Nachweis von Darmkrebszellen, die in Blut eines Gesunden inokuliert wurden. Hier zeigt sich unmittelbar, daß der Einsatz einer Kombination der Antikörper Ber-Ep 4 und MOC-31 zu einer verbesserten Empfindlichkeit bezüglich des mRNS-Markers EGF-R führt. Beim Einsatz der beiden Antikörper gleichzeitig wird eine Nachweisempfindlichkeit von 100 Zellen pro Milliliter Blut erreicht, während bei Einsatz lediglich eines Antikörpers die Empfindlichkeit bei ca. 1000 Zellen pro Milliliter Blut liegt.

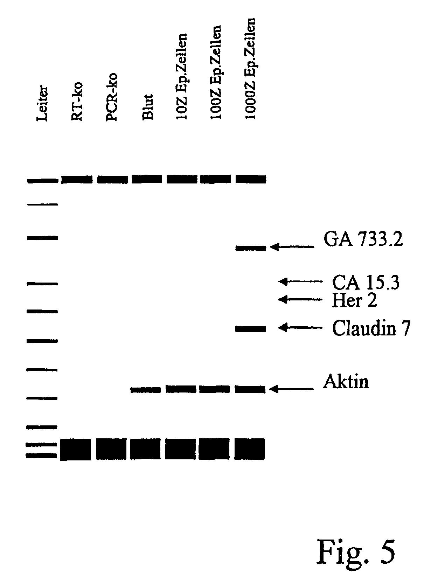

Figur 5 zeigt einen Versuch, bei dem mittels einer Antikörperkombination von Ber-Ep 4, HMPV.2 und GP1.4, die mit zumindest einem der Antikörper markierten Zellen selektioniert wurden und anschließend auf die mRNS-Marker GA 733.2, CA 15.3, Her 2 und Claudin 7 untersucht wurden. Allerdings wurde hier dem Blut der Gesunden keine Tumorzelle zugegeben sondern definierte Mengen von Epithelialzellen. Wie aus Figur 5 unmittelbar hervorgeht, zeigen lediglich zwei der Marker bei einer sehr hohen Zahl von epithelialen Zellen ein positives Ergebnis.

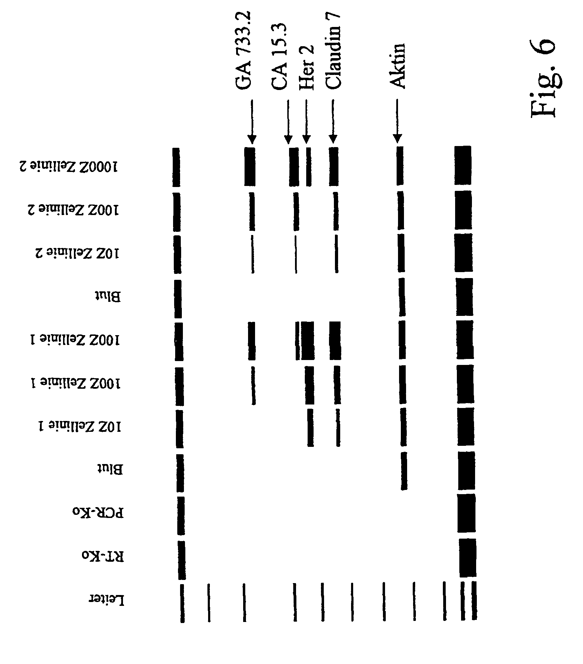

In Figur 6 wurden Tumorzellen zweier verschiedener Brustkrebszellinien (MCF-7 und SKBR-3)einer Blutprobe zugegeben. Als Antikörper wurden die Antikörper Ber-Ep 4, HMPV.2 und GP1.4 in Kombination eingesetzt. Wie unmittelbar zu erkennen ist, wird durch den Einsatz der vier mRNS-Marker GA 733.2, CA 15.3, Her 2 und Claudin 7 jeweils gesichert bis herab zu 10 Zellen der einzelnen Zellinien pro Milliliter eine Erkennung der Brustkrebszellen gewährleistet. Eine unspezifische Reaktion in Blut ohne Brustkrebszellen trat nicht auf. Allerdings sind die Tumormarker GA 733.2 und CA 15.3 für die Zellinie 2 (SKBR-3) sensitiver, während der Tumormarker Her 2 für die Zellinie 1 (MCF-7) sensitiver ist. So können dann auch die einzelnen Untertypen von Brustkrebszellen anhand des aufgetretenen Markermusters voneinander unterschieden werden.

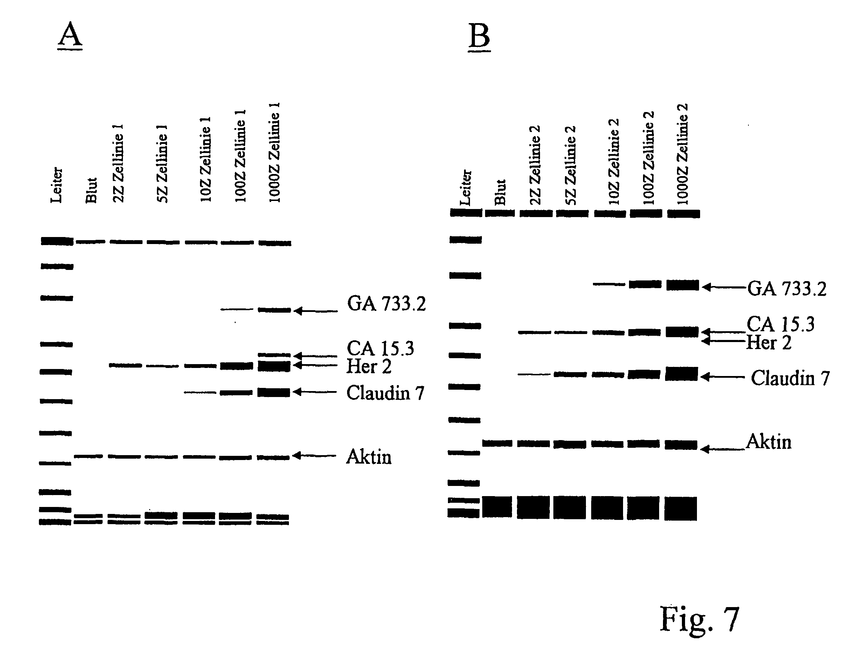

Auch in Figur 7 sind Brustkrebszellen verschiedener Zellinien (MCF-7) in Figur 7A und SKBR 3 in Figur 7B in Blut inokuliert worden. Als Antikörper zur Selektion der Zellen aus der Blutprobe wurden die Antikörper Ber-Ep 4, HMPV.2 und GPl.4 in Kombination verwendet. Es ist unmittelbar zu erkennen, daß bei Einsatz einer Kombination der mRNA-Marker GA 733.2, CA 15.3 Her 2 und Claudin 7 in jedem Falle mindestens einer der Marker bis herab zu 2 Zellen pro 5 Milliliter positiv reagiert, ohne daß das Blut ohne Tumorzellen einen Expressionshintergrund liefern würde. Auch hier ist wieder ein differenzielles Ansprechverhalten der beiden Zellinien auf die vier unterschiedlichen mRNS-Marker zu erkennen.

Befinden sich beispielsweise jedoch in einer Blutprobe beide Zellinien, so wäre durch die gewählt Markerkombination in jedem Falle bis herab von 2 Zellen pro 5 Milliliter Blut eine Erkennung beider Zellinien gewährleistet, d. h. der Nachweis von Brustkrebszellen im Blut wäre unabhängig vom Zelltyp der Brustkrebszellinie mit hoher Sensitivität möglich.

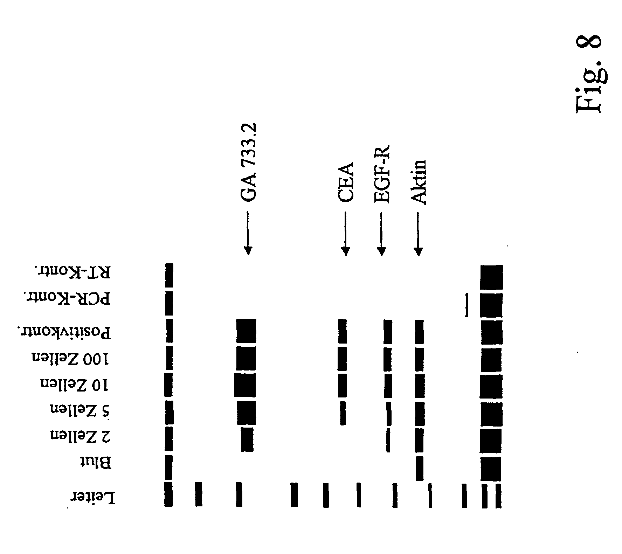

Figur 8 zeigt den Nachweis von Darmkrebszellen, die in Blut inokuliert wurden. Dabei erfolgte die Selektion der Zellen mit den zwei Antikörpern Ber-Ep 4 und MOC-31. Der molekularbiologische Nachweisschritt erfolgte mit den mRNS-Markern GA 733.2, CEA und EGF-R. Zwei Tumorzellen waren in 5 Milliliter Blut nachweisbar.

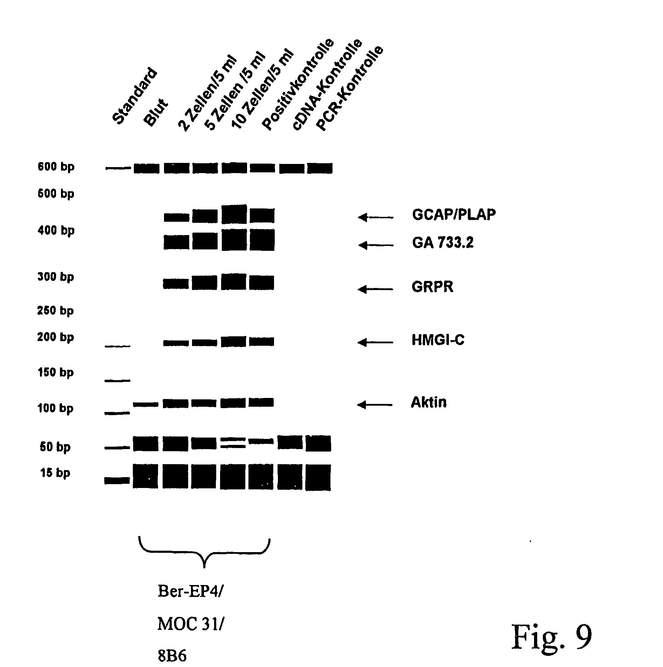

Figur 9 zeigt wiederum eine Messung mit einer Kombination aus drei Antikörpern Ber-Ep 4 , MOC-31 und 8B6 sowie den mRNA-Markern GCAP/PLAP, GA 733.2, GRPR und HMGI-C an Blut, in das Hodenkrebszellen inokuliert wurden.

Mit jedem der molekularbiologischen Marker gelingt bei Einsatz dieser Dreierkombination von Antikörpern für die Zellselektionierung im immunologischen Selektionierungsschritt der Nachweis bis herab zu 2 Zellen pro 5 Milliliter.

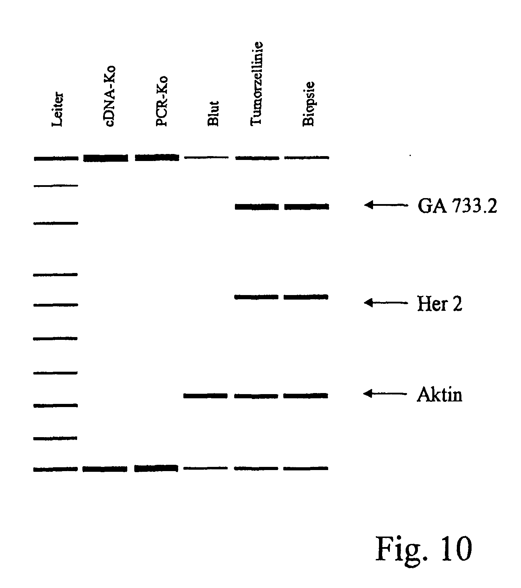

Figur 10 zeigt abschließend die Abtrennung von seltenen Zellen aus dem Zellgemisch eines Biopsiematerials. Hierzu wurden Biopsiematerial aus Brustgewebe, das Tumorgewebe mit einem vermuteten Primärtumor enthielt, mechanisch vereinzelt und über Gaze von Zelltrümmern, Bindegewebe etc. getrennt. Das gewonnene Zellgemisch, das sowohl Zellen des vermuteten Tumorgewebes als auch Zellen des umliegenden gesunden Gewebes enthielt, wurde einer Zellselektion mit einem an eine Festphase gekoppelten Antikörpergemisch (Magnetpartikel mit Antikörpern GP1.4, HMPV.2 und Ber-Ep 4) versetzt und nach Inkubation zur Herstellung der Antigen-Antikörperbindung magnetisch separiert. Anschließend erfolgte ein mRNS-Nachweis bezüglich der Marker GA 733.2 und Her 2. Als Kontrolle wurde zugleich eine Zellinie eines Brustkrebses parallel ebenfalls bestimmt. Wie zu erkennen ist, erfolgt ein positiver Nachweis, daß die Biopsie tatsächlich einen Brustkrebs-Tumor enthielt.

Die Banden, die in den Figuren 8 und 9 mit "Positivkontrolle" gekennzeichnet sind, zeigen Ergebnisse von Proben mit der Zellinien HT 29 für Darmtumor (Fig. 8) bzw. Tera/l für Hodentumor (Fig. 9).

## Patentansprüche

1. Verfahren zum qualitativen und/oder quantitativen Nachweis von vorbestimmten biologischen Zellen aus bzw. in einer biologische Zellen enthaltenden Probe, wobei
die Probe mit einer vorbestimmten Kombination von mindestens zwei Antikörpern und/oder Antikörperderivaten, die mit ihren Bindungsstellen an verschiedene auf den nachzuweisenden Zellen präferentiell vorhandene Epitope binden, und/oder mit mindestens einem bispezifischen Antikörper und/oder Antikörperderivat, der/das mit seinen beiden Bindungsstellen an unterschiedliche auf den nachzuweisenden Zellen präferentiell vorhandene Epitope bindet, versetzt wird,
die mit mindestens einem der Antikörper und/oder Antikörperderivate markierten Zellen aus der Probe abgetrennt werden,
und die abgetrennten Zellen mit einer vorbestimmten Kombination mindestens zweier molekularbiologischer Nachweisreagentien auf die Expression einer vorbestimmten Kombination mindestens zweier mRNA-Abschnitte geprüft werden, deren Expression in den nachzuweisenden Zellen präferentiell erfolgt, und
erfasst wird, ob zumindest einer der mRNA-Abschnitte exprimiert ist.

2. Verfahren nach dem vorhergehenden Anspruch **dadurch gekennzeichnet, dass** die Abtrennung der markierten Zellen in Flüssigphase oder Festphase erfolgt.

3. Verfahren nach einem der vorhergehenden Ansprüche, **dadurch gekennzeichnet, dass** auf Festphasen gekoppelte Antikörper oder Antikörperderivate verwendet werden, um die Zielzellen aus der Probe abzutrennen.

4. Verfahren nach einem der vorhergehenden Ansprüche, **dadurch gekennzeichnet, dass** mit Fluorophoren markierte Antikörper oder Antikörperderivate verwendet werden und die Abtrennung der markierten Zellen aus der Probe mittels Durchflußzytometrie (fluoreszenzassoziierte Zelltrennung, FACS) erfolgt..

5. Verfahren nach einem der vorhergehenden Ansprüche, **dadurch gekennzeichnet; dass** mit magnetischen bzw. pseudomagnetischen Partikeln gekoppelte Antikörper oder Antikörperderivate verwendet werden und zur Abtrennung der markierten Zellen aus der Probe die magnetischen bzw. pseudomagnetischen, antikörpergekoppelten Partikel nach der Mischung mit der Probe magnetisch von der Probe getrennt werden.

6. Verfahren nach einem der vorhergehenden Ansprüche, **dadurch gekennzeichnet, dass** die Antikörper oder Antikörperderivate Bindungsstellen aufweisen, die an Tumorzellen binden.

7. Verfahren nach einem der vorhergehenden Ansprüche, **dadurch gekennzeichnet, dass** die Antikörper oder Antikörperderivate Bindungsstellen aufweisen, die an Zellen eines oder mehrerer bestimmter Tumortypen oder -untertypen binden.

8. Verfahren nach einem der vorhergehenden Ansprüche, **dadurch gekennzeichnet, dass** zur Abtrennung von Tumorzellen oder Zellen eines bestimmten Tumortyps oder -subtyps die Antikörper oder Antikörperderivate Bindungsstellen aufweisen, die an Epitope eines epithelialen Antigens, eines epithelialen Membranantigens, des Antigens MUC1 und/oder des Antigens PLAP binden.

9. Verfahren nach einem der vorhergehenden Ansprüche, **dadurch gekennzeichnet, dass** mindestens einer der Antikörper GP1.4, MOC-31, Ber-EP4, HMPV.2, 8B6, E29 und/oder 131-11741 verwendet wird.

10. Verfahren nach einem der vorhergehenden Ansprüche, **dadurch gekennzeichnet, dass** zur Abtrennung von Tumorzellen allgemein oder eines bestimmten Typs oder Subtyps eine Kombination von Antikörpern verwendet wird, die die Antikörper Ber-EP4 und M0C31 oder mindestens zwei der Antikörper HMPV.2, GP1.4 und Ber-EP4 enthält.

11. Verfahren nach einem der vorhergehenden Ansprüche, **dadurch gekennzeichnet, dass** zur Abtrennung von Brusttumorzellen eine Kombination von Antikörpern verwendet wird, die mindestens zwei der Antikörper 131-11741, GP1.4, E29 und HMPV.2 oder mindestens zwei der Antikörper HIKEIV.2, GP1.4 und Ber-EP4 enthält.

12. Verfahren nach einem der vorhergehenden Ansprüche, **dadurch gekennzeichnet, dass** zur Abtrennung von Darmtumorzellen eine Kombination von Antikörpern verwendet wird, die die Antikörper Ber-EP4 und MOC-31 enthält.

13. Verfahren nach einem der vorhergehenden Ansprüche, **dadurch gekennzeichnet, dass** zur Abtrennung von Hodentumorzellen eine Kombination von Antikörpern verwendet wird, die mindestens zwei der Antikörper MOC31, Ber-EP4 und 8B6 enthält.

14. Verfahren nach einem der vorhergehenden Ansprüche, **dadurch gekennzeichnet, dass** zum Nachweis von Tumorzellen oder Zellen eines bestimmten Tumortyps oder -subtyps eine Kombination von mRNS-Abschnitten geprüft wird, die mRNS-Abschnitte korrespondierend zu Sequenzabschnitten mindestens zwei der Gene GA733.2, EGFR, CEA, HER2/neu, Claudin-7 (CLDN7), GCAP(ALPPL2)/ALPP, GRPR, HMGIC, CK20, MAGE3, MUC1 und Stanniocalcin (STC1) enthält.

15. Verfahren nach dem vorhergehenden Anspruch, **dadurch gekennzeichnet, dass** zum Nachweis von Tumorzellen oder Zellen eines bestimmten Tumortyps oder -subtyps eine Kombination von mRNS-Abschnitten geprüft wird, die mRNS-Abschnitte korrespondierend zu Sequenzabschnitten mindestens zwei der Gene EGFR, GA733.2 und HER-2/NEU enthält.

16. Verfahren nach Anspruch 14, **dadurch gekennzeichnet, dass** zum Nachweis von Brusttumorzellen eine Kombination von mRNS-Abschnitten verwendet wird, die mRNS-Abschnitte korrespondierend zu Sequenzabschnitten mindestens zwei der Gene GA733.2, MUC1, Her-2/neu, Claudin7, CK20, PIAGE3, Stanniocalcin, EGFR und CEA enthält.

17. Verfahren Anspruch 16, **dadurch gekennzeichnet, dass** zum Nachweis von Brusttumorzellen eine Kombination von mRNS-Abschnitten verwendet wird, die mRNS-Abschnitte korrespondierend zu Sequenzabschnitten der beiden Gene GA733.12 und MUC1, korrespondierenden zu Sequenzabschnitten der beiden Gene Her-2/neu und Claudin7, korrespondierend zu Sequenzabschnitten mindestens zwei der Gene CK20, MAGE-3 und MUC1 und/oder korrespondierend zu Sequenzabschnitten mindestens zwei der Gene Stanniocalcin, EGFR und CEA enthält.

18. Verfahren nach Anspruch 14, **dadurch gekennzeichnet, dass** zum Nachweis von Darmtumorzellen eine Kombination von mRNS-Abschnitten verwendet wird, die mRNS-Abschnitte korrespondierend zu Sequenzabschnitten mindestens zwei der Gene CK20, EGFR, GA733.2, CEA und Stanniocalcin enthält.

19. Verfahren nach Anspruch 18, **dadurch gekennzeichnet, dass** zum Nachweis von Darmtumorzellen eine Kombination von mRNS-Abschnitten verwendet wird, die mRNS-Abschnitte korrespondierend zu Sequenzabschnitten mindestens zwei der Gene CK20, EGFR, CEA und Stanniocalcin und/oder korrespondierend zu Sequenzabschnitten mindestens zwei der Gene EGFR, CEA und GA733.2 enthält.

20. Verfahren nach Anspruch 14, **dadurch gekennzeichnet, dass** zum Nachweis von Hodentumorzellen eine Kombination von mRNS-Abschnitten verwendet wird, die mRNS-Abschnitte korrespondierend zu Sequenzabschnitten mindestens zwei der Gene ALPP/ALPPL2 (GCAP), CGA733.2(=EGP-40), HMGI-C, GRPR enthält.

21. Verfahren, nach einem der vorhergehenden Ansprüche, **dadurch gekennzeichnet, dass** die mRNS-Abschnitte unter Verwendung von Polymerasekettenreaktion (PCR), LCR, NASBA RT-PCR und/oder Hybridisierungsverfahren vervielfältigt und/oder nachgewiesen werden.

22. Verfahren nach einem der vorhergehenden Ansprüche, **dadurch gekennzeichnet, dass** die mRNS der abgetrennten Zellen in cDNS revers transkribiert, die cDNS vervielfältigt und anschließend das Vorhandensein oder Fehlen des nachzuweisenden mRNS-Abschnittes erfasst wird.

23. Verfahren nach dem vorhergehenden Anspruch, **dadurch gekennzeichnet, dass** die vervielfältigte cDNS durch geeignete Restriktionsenzyme verdaut und anhand der erzeugten cDNS-Bruchstücke das Vorhandensein oder Fehlen der nachzuweisenden mRNS erfasst wird (Fragmentanalyse).

24. Verfahren nach einem der beiden vorhergehenden Ansprüche, **dadurch gekennzeichnet, dass** die der nachzuweisenden mRNS entsprechende cDNS mittels fluoreszenzbasierter Echtzeit -PCR bestimmt wird.

25. Verfahren nach einem der vorhergehenden Ansprüche, **dadurch gekennzeichnet, dass** als interne Kontrolle die mRNS des Proteins β-Aktin erfasst wird.

26. Verwendung eines Verfahrens nach einem der vorhergehenden Ansprüche zum Nachweis von Zellen seltenen Typs in Suspensionen und Zellmischungen, insbesondere von Tumorzellen, Epithelzellen und/oder Endothelzellen in Körperflüssigkeiten, peripherem Blut, Sputum, Ascites, Lymphe, Urin, Knochenmark und/oder Biopsiematerial und/oder von fötalen Zellen in Amnionflüssigkeit oder maternalem peripherem Blut.

27. Verwendung nach dem vorhergehenden Anspruch zur Diagnostik und/oder Behandlungskontrolle von Tumorerkrankungen.

28. Verwendung nach dem vorhergehenden Anspruch zur Diagnostik und/oder Behandlungskontrolle von Hodentumor, Brusttumor und/oder Darmtumor.

## Claims

1. Method for qualitative and/or quantitative detection of predetermined biological cells from or in a sample containing biological cells, wherein
the sample is mixed with a predetermined combination of at least two antibodies and/or antibody derivatives, which bind with their binding sites to different epitopes which are preferentially present on the cells to be detected,
and/or with at least one bispecific antibody and/or antibody derivative, which binds with its two binding sites to different epitopes which are preferentially present on the cells to be detected,
the cells marked with at least one of the antibodies and/or antibody derivatives are separated from the sample,
and the separated cells are tested with a predetermined combination of at least two molecular-biological detection reagents for the expression of a predetermined combination of at least two mRNA portions, the expression of which is effected preferentially in the cells to be detected, and
it is detected whether at least one of the mRNA portions is expressed.

2. Method according to the preceding claim, **characterised in that** the separation of the marked cells is effected in liquid phase or solid phase.

3. Method according to one of the preceding claims, **characterised in that** antibodies or antibody derivatives coupled to solid phases are used in order to separate the target cells from the sample.

4. Method according to one of the preceding claims, **characterised in that** antibodies or antibody derivatives marked with fluorophores are used and the separation of the marked cells from the sample is effected by means of flow cytometry (fluorescence-associated cell separation, FACS).

5. Method according to one of the preceding claims, **characterised in that** antibodies or antibody derivatives coupled to magnetic or pseudo-magnetic particles are used and in order to separate the marked cells from the sample, the magnetic or pseudo-magnetic antibody-coupled particles after mixing with the sample are separated magnetically from the sample.

6. Method according to one of the preceding claims, **characterised in that** the antibodies or antibody derivatives have binding sites which bind to tumour cells.

7. Method according to one of the preceding claims, **characterised in that** the antibodies or antibody derivatives have binding sites which bind to cells of one or more specific tumour types or sub-types.

8. Method according to one of the preceding claims, **characterised in that**, in order to separate tumour cells or cells of a specific tumour type or sub-type, the antibodies or antibody derivatives have binding sites which bind to epitopes of an epithelial antigen, of an epithelial membrane antigen, of the antigen MUC1 and/or of the antigen PLAP.

9. Method according to one of the preceding claims, **characterised in that** at least one of the antibodies GP1.4, MOC-31, Ber-EP 4, HMPV.2, 8B6, E29 and/or 131-11741 is used.

10. Method according to one of the preceding claims, **characterised in that**, in order to separate tumour cells in general or of a specific type or sub-type, a combination of antibodies is used, which contains the antibodies Ber-EP4 and MOC31 or at least two of the antibodies HMPV.2, GP1.4 and Ber-EP4.

11. Method according to one of the preceding claims, **characterised in that**, in order to separate breast tumour cells, a combination of antibodies is used, which contains at least two of the antibodies 131-11741, GPl.4, E29 and HMPV.2 or at least two of the antibodies HMEIV.2, GPl.4 and Ber-EP4.

12. Method according to one of the preceding claims, **characterised in that**, in order to separate colon tumour cells, a combination of antibodies is used, which contains the antibodies Ber-EP4 and MOC-31.

13. Method according to one of the preceding claims, **characterised in that**, in order to separate testicular tumour cells, a combination of antibodies is used, which contains at least two of the antibodies MOC31, Ber-EP4 and 8B6.

14. Method according to one of the preceding claims, **characterised in that**, in order to detect tumour cells or cells of a specific tumour type or sub-type, a combination of mRNA portions is tested, which contains mRNA portions corresponding to sequence portions of at least two of the genes GA733.2, EGFR, CEA, HER2/neu, claudin-7 (CLDN7), GCAP (ALPPL2)/ALPP, GRPR, HMGIC, CK20, MAGE3, MUC1 and stanniocalcin (STC1).

15. Method according to the preceding claim, **characterised in that**, in order to detect tumour cells or cells of a specific tumour type or sub-type, a combination of mRNA portions is tested, which contains mRNA portions corresponding to sequence portions of at least two of the genes EGFR, GA733.2 and HER-2/NEU.

16. Method according to claim 14, **characterised in that**, in order to detect breast tumour cells, a combination of mRNA portions is used, which contains mRNA portions corresponding to sequence portions of at least two of the genes GA733.2, MUC1, Her-2/neu, claudin7, CK20, PIAGE3, stanniocalcin, EGFR and CEA.

17. Method according to claim 16, **characterised in that**, in order to detect breast tumour cells, a combination of mRNA portions is used, which contains mRNA portions corresponding to sequence portions of both genes GA733.12 and MUC1, corresponding to sequence portions of both genes Her-2/neu and claudin7, corresponding to sequence portions of at least two of the genes CK20, MAGE-3 and MUC1 and/or corresponding to sequence portions of at least two of the genes stanniocalcin, EGFR and CEA.

18. Method according to claim 14, **characterised in that**, in order to detect colon tumour cells, a combination of mRNA portions is used, which contains mRNA portions corresponding to sequence portions of at least two of the genes CK20, EGFR, GA733.2, CEA and stanniocalcin.

19. Method according to claim 18, **characterised in that**, in order to detect colon tumour cells, a combination of mRNA portions is used, which contains mRNA portions corresponding to sequence portions of at least two of the genes CK20, EGFR, CEA and stanniocalcin and/or corresponding to sequence portions of at least two of the genes EGFR, CEA and GA733.2.

20. Method according to claim 14, **characterised in that**, in order to detect testicular tumour cells, a combination of mRNA portions is used, which contains mRNA portions corresponding to sequence portions of at least two of the genes ALPP/ALPPL2 (GCAP), CGA733.2 (=EGP-40), HMGI-C, GRPR.

21. Method according to one of the preceding claims, **characterised in that** the mRNA portions are multiplied and/or detected using polymerase chain reaction (PCR), LCR, NASBA RT-PCR and/or hybridisation methods.

22. Method according to one of the preceding claims, **characterised in that** the mRNA of the separated cells is transcribed reversely into cDNA, the cDNA is multiplied and subsequently the presence or absence of the mRNA portion to be detected is detected.

23. Method according to the preceding claim, **characterised in that** the multiplied cDNA is digested by suitable restriction enzymes and the presence or absence of the mRNA to be detected is detected by means of the produced cDNA fragments (fragment analysis).

24. Method according to one of the two preceding claims, **characterised in that** the cDNA corresponding to the mRNA to be detected is determined by means of fluorescence-based real time-PCR.

25. Method according to one of the preceding claims, **characterised in that** the mRNA of the protein β-actin is detected as internal control.

26. Use of a method according to one of the preceding claims for detecting cells of an uncommon type in suspensions and cell mixtures, in particular tumour cells, epithelial cells and/or endothelial cells, in body fluids, peripheral blood, sputum, ascites, lymph, urine, bone marrow and/or biopsy material and/or foetal cells in amniotic fluid or maternal peripheral blood.

27. Use according to the preceding claim for diagnostics and/or treatment control of tumour diseases.

28. Use according to the preceding claim for diagnostics and/or treatment control of testicular tumour, breast tumour and/or colon tumour.

## Revendications

1. Procédé de détection qualitative et/ou quantitative de cellules biologiques prédéterminées dans un échantillon contenant des cellules biologiques, dans lequel l'échantillon est mélangé à une combinaison prédéterminée d'au moins deux anticorps et/ou dérivés d'anticorps, qui se fixent, par leurs points de fixation, sur divers épitopes présents préférentiellement sur les cellules à détecter, et/ou à au moins un anticorps et/ou un dérivé d'anticorps bispécifique, qui se fixe, par ses deux points de fixation, sur différents épitopes présents préférentiellement sur les cellules à détecter,
les cellules marquées par au moins un des anticorps et/ou des dérivés d'anticorps sont séparées de l'échantillon,
et les cellules séparées sont testées avec une combinaison prédéterminée d'au moins deux réactifs de détection de biologie moléculaire pour déceler l'expression d'une combinaison prédéterminée d'au moins deux segments d'ARNm dont l'expression s'effectue de préférence dans les cellules à détecter, et
on détecte si au moins un des segments d'ARNm est exprimé.

2. Procédé selon la revendication précédente, **caractérisé en ce que** la séparation des cellules marquées s'effectue en phase liquide ou en phase solide.

3. Procédé selon l'une quelconque des revendications précédentes, **caractérisé en ce qu'**on utilise des anticorps ou des dérivés d'anticorps couplés à des phases solides pour séparer les cellules cibles de l'échantillon.

4. Procédé selon l'une quelconque des revendications précédentes, **caractérisé en ce qu'**on utilise des anticorps ou des dérivés d'anticorps marqués avec des fluorophores et **en ce que** la séparation des cellules marquées de l'échantillon s'effectue par cytométrie débitmétrique (séparation cellulaire associée à la fluorescence, FACS).

5. Procédé selon l'une quelconque des revendications précédentes, **caractérisé en ce qu'**on utilise des anticorps ou des dérivés d'anticorps couplés avec des particules magnétiques ou pseudomagnétiques, et
**en ce que**, pour séparer les cellules marquées de l'échantillon, on sépare magnétiquement de l'échantillon les particules magnétiques ou pseudomagnétiques couplées avec les anticorps après mélange avec l'échantillon.

6. Procédé selon l'une quelconque des revendications précédentes, **caractérisé en ce que** les anticorps ou les dérivés d'anticorps présentent des points de fixation qui se fixent sur des cellules tumorales.

7. Procédé selon l'une quelconque des revendications précédentes, **caractérisé en ce que** les anticorps ou les dérivés d'anticorps présentent des points de fixation qui se fixent sur des cellules d'un ou plusieurs types ou sous-types déterminés de tumeur.

8. Procédé selon l'une quelconque des revendications précédentes, **caractérisé en ce que**, pour séparer des cellules tumorales ou des cellules d'un type ou d'un sous-type déterminé de tumeur, les anticorps ou les dérivés d'anticorps présentent des points de fixation qui se fixent sur des épitopes d'un antigène épithélial, d'un antigène membranaire épithélial, de l'antigène MUC1 et/ou de l'antigène PLAP.

9. Procédé selon l'une quelconque des revendications précédentes, **caractérisé en ce qu'**on utilise au moins un des anticorps GP1.4, MOC-31, Ber-EP4, HMPV.2, 8B6, E29 et/ou 131-11741.

10. Procédé selon l'une quelconque des revendications précédentes, **caractérisé en ce que**, pour séparer des cellules tumorales de manière générale ou d'un type ou d'un sous-type déterminé, on utilise une combinaison d'anticorps qui contient les anticorps Ber-EP4 et M0C31 ou au moins deux des anticorps HMPV.2, GP1.4 et Ber-EP4.

11. Procédé selon l'une quelconque des revendications précédentes, **caractérisé en ce**, pour séparer des cellules de tumeurs du sein, on utilise une combinaison d'anticorps qui contient au moins deux des anticorps 131-11741, GP1.4, E29 et HMPV.2 ou au moins deux des anticorps HMEIV.2, GP1.4 et Ber-EP4.

12. Procédé selon l'une quelconque des revendications précédentes, **caractérisé en ce que**, pour séparer des cellules de tumeur intestinale, on utilise une combinaison d'anticorps qui contient les anticorps Ber-EP4 et MOC-31.

13. Procédé selon l'une quelconque des revendications précédentes, **caractérisé en ce que**, pour séparer des cellules de tumeurs testiculaires, on utilise une combinaison d'anticorps qui contient au moins deux des anticorps MOC31, Ber-EP4 et 8B6.

14. Procédé selon l'une quelconque des revendications précédentes, **caractérisé en ce que**, pour détecter des cellules tumorales ou des cellules d'un type ou sous-type prédéterminé de tumeur, on teste une combinaison de segments d'ARNm qui contient des segments d'ARNm correspondant à des segments de séquence d'au moins deux des gènes GA733.2, EGFR, CEA, HER2/neu, Claudin-7 (CLDN7), GCAP(ALPPL2)/ALPP, GRPR, HMGIC, CK20, MAGE3, MUC1 et stanniocalcine (STC1).

15. Procédé selon la revendication précédente, **caractérisé en ce que**, pour détecter des cellules tumorales ou des cellules d'un type ou d'un sous-type déterminé de tumeur, on teste une combinaison de segments d'ARNm qui contient des segments d'ARNm correspondant à des segments de séquence d'au moins deux des gènes EGFR, GA733.2 et HER-2/NEU.

16. Procédé selon la revendication 4, **caractérisé en ce que**, pour détecter des cellules tumorales du sein, on emploie une combinaison de segments d'ARNm qui contient des segments d'ARNm correspondant à des segments de séquence d'au moins deux des gènes GA733.2, MUC1, Her-2/neu, Claudin7, CK20, PIAGE3, stanniocalcine, EGFR et CEA.

17. Procédé selon la revendication 16, **caractérisé en ce que**, pour détecter des cellules tumorales du sein, on utilise une combinaison de segments d'ARNm qui contient des segments d'ARNm correspondant à des segments de séquence des deux gènes GA733.12 et MUC1, correspondant à des segments de séquence des deux gènes Her-2/neu et Claudin7, correspondant à des segments de séquence d'au moins deux des gènes CK20, MAGE-3 et MUC1 et/ou correspondant à des segments de séquence d'au moins deux des gènes stanniocalcine, EGFR et CEA.

18. Procédé selon la revendication 14, **caractérisé en ce que**, pour détecter des cellules de tumeurs intestinales, on emploie une combinaison de segments d'ARNm qui contient des segments d'ARNm correspondant à des segments de séquence d'au moins deux des gènes CK20, EGFR, GA733.2, CEA et stanniocalcine.

19. Procédé selon la revendication 18, **caractérisé en ce que**, pour détecter des cellules de tumeurs intestinales, on utilise une combinaison de segments d'ARNm qui contient des segments d'ARNm correspondant à des segments de séquence d'au moins deux des gènes CK20, EGFR, CEA et stanniocalcine et/ou correspondant à des segments de séquence d'au moins deux des gènes EGFR, CEA et GA733.2.

20. Procédé selon la revendication 14, **caractérisé en ce que**, pour détecter des cellules de tumeurs testiculaires, on emploie une combinaison de segments d'ARNm qui contient des segments d'ARNm correspondant à des segments de séquence d'au moins deux des gènes ALPP/ALPPL2 (GCAP), CGA733.2 (=EGP-40), HMGI-C et GRPR.

21. Procédé selon l'une quelconque des revendications précédentes, **caractérisé en ce que** l'on réplique et/ou détecte les segments d'ARNm en utilisant une amplification en chaîne par polymérase (PCR), LCR, NASBA RT-PCR et/ou un procédé d'hybridation.

22. Procédé selon l'une quelconque des revendications précédentes, **caractérisé en ce que** les ARNm des cellules séparées sont transcrits à l'inverse dans des ADNc et les ADNc sont répliqués et, ensuite, on décèle la présence ou l'absence du segment d'ARNm à détecter.

23. Procédé selon la revendication précédente, **caractérisé en ce que** l'ADNc répliqué est digéré par des enzymes de restriction appropriées et la présence ou l'absence des ARNm à détecter est décelée à l'aide des fragments d'ADNc produits (analyse fragmentaire).

24. Procédé selon l'une quelconque des deux revendications précédentes, **caractérisé en ce que** l'ADNc correspondant à l'ARNm à détecter est déterminé au moyen d'une PCR en temps réel à base fluorescente.

25. Procédé selon l'une quelconque des revendications précédentes, **caractérisé en ce qu'**on décèle comme témoin interne l'ARNm de la protéine β-actine.

26. Utilisation d'un procédé selon l'une quelconque des revendications précédentes pour détecter des cellules d'un type rare dans des suspensions et des mélanges cellulaires, en particulier de cellules tumorales, de cellules épithéliales et/ou de cellules endothéliales dans des liquides corporels, le sang périphérique, des expectorations, des ascites, de la lymphe, de l'urine, la moelle osseuse et/ou un matériau biopsique et/ou des cellules foetales dans le liquide amniotique ou le sang périphérique maternel.

27. Utilisation selon la revendication précédente pour le diagnostic et/ou le contrôle de traitement de maladies tumorales.

28. Utilisation selon la revendication précédente pour le diagnostic et/ou le contrôle de traitement de tumeurs testiculaires, de tumeurs du sein et/ou de tumeurs intestinales.
